# EUROPEAN PATENT SPECIFICATION

(11) **EP 1 375 660 B1**
(45) Date of publication and mention of the grant of the patent: **09.02.2005**
(21) Application number: 03011621.4
(22) Date of filing: 22.05.2003
(51) Int. Cl.: C12N 15/10, C12Q 1/68, C12P 19/34

(54) **Method of isolating nucleic acid**
Verfahren für die Nukleinsäureisolierung
Procédé pour l'isolation d'acides nucléiques

(30) Priority: 24.05.2002 JP 2002150836
(43) Date of publication of application: 02.01.2004
(73) Proprietor: Tosoh Corporation, Shunan-shi Yamaguchi-ken 746-8501 (JP)
(72) Inventor: Saitoh, Juichi, Yamato-shi, Kanagawa-ken (JP); Kurihara, Yoshifumi, Ebina-shi, Kanagawa-ken (JP); Ishiguro, Takahiko, Yokohama-shi, Kanagawa-ken (JP)
(74) Representative: Leson, Thomas Johannes Alois, Dipl.-Ing.

(56) References cited:
- WO-A-97/34909
- WO-A-98/14460
- US-A- 5 637 687

### SEQUENCE LISPING

<110> Tosoh Corporation
<120> METHOD OF ISOLATING NUCLEIC ACID
<130> PA211-0772/EP 38557
<140> EP 03011621.4-2401
   <141> 2003-05-22
<150> JPA 2002150836
   <151> 2002-05-24
<160> 2
<170> PatentIn version 3.1
<210> 1
   <211> 20
   <212> DNA
   <213> Artificial sequence
<220>
   <223> Primer (R)
   <400> 1
   gcctttcgcg acccaacact 20
<210> 2
   <211> 22
   <212> DNA
   <213> Artificial sequence
<220>
   <223> Primer (F) <400> 2
   <400> 2
   acactccacc atagatcact cc 22

## Description

The present invention relates to a method of isolating nucleic acid from other components in a sample.

Isolation (preparation) of nucleic acid from samples containing nucleic acid is an important operation in the fields of biotechnology and clinical diagnosis. For example, genetic tests involve isolation of cellular or viral nucleic acid from other components to give a nucleic acid isolate to be used in the subsequent nucleic acid amplification or detection step.

Conventional techniques for nucleic acid isolation include the protease K/phenol method using deleterious phenol or chloroform (Sambrook, J et al, Molecular Cloning, A Laboratory Manual, 2nd ed., Cold Spring Harbor Laboratory Press (1989) 7.12-7.15) and the AGPC method (Chomczunski, P. and Sacchi, N., Analytical Biochemistry (1987) 162, 156-159). In addition, another method is disclosed in JP-A-7-59572 for nucleic acid isolation without using phenol or chloroform and comprises (1) a step of mixing a sample and at least one carrier selected from the group consisting of dextran, acrylamide and carboxymethylcellulose into a liquid mixture, (2) a step of adding a reagent C comprising a reagent A selected from the group consisting of guanidine thiocyanate, guanidine hydrochloride, potassium thiocyanate and sodium thiocyanate and at least one reagent B selected from the group consisting of n-propyl alcohol, isopropyl alcohol, n-butyl alcohol, sec-butyl alcohol, tert-butyl alcohol and tert-amyl alcohol, to the liquid mixture obtained in the step (1) to insolubilize the nucleic acid and the carrier, and (3) a step of separating the insolubilized nucleic acid and carrier from the liquid phase.

Among the above-mentioned conventional techniques, the protease K/phenol method and the AGPC method have problems in operational safety and disposal of used reagents because of the use of deleterious phenol or chloroform. These techniques also require high-speed centrifugation to separate nucleic acid and has a problem of the need for a special instrument.

Though the method disclosed in JP-A-7-59572 does not require deleterious phenol or chloroform and does not require as difficult an operation as high-speed centrifugation to separate nucleic acid from the liquid phase, it still has a problem that filtration or centrifugation is necessary to separate the insolubilized nucleic acid and carrier from the liquid phase.

As described above, conventional techniques for nucleic acid isolation have the problems of complex operations and the need for special instruments. Due to the complex operations, the conventional techniques are difficult to automate and require skills to isolate and prepare nucleic acid quickly and reproductively from numerous samples without nucleic acid carryover between samples or between samples and the environment.

The object of the present invention is to provide a method of isolating (preparing) nucleic acid which neither uses deleterious substances such as phenol and chloroform nor requires a special instrument such as filtration and centrifugation, and therefore is easy to automate.

The problem with conventional methods of isolating and preparing nucleic acid resides in how to simplify separation of nucleic acid from crude extracts containing protein and other contaminants. Isolation of protein based on partition between two aqueous phases has been attempted recently as an isolation technique which does not involve filter or centrifugal separation. However, no specific conditions have been reported so far for isolation and preparation of nucleic acid from crude extracts containing protein based on partition between two aqueous phases.

The present inventors have conducted extensive research on isolation of nucleic acid based on partition between two aqueous phases and accomplished the present invention. The present invention has been accomplished to attain the above-mentioned object. The invention as defined in Claim 1 of the present application provides a method of isolating nucleic acid in a sample, which comprises preparing an aqueous solution comprising the sample, a first water-soluble polymer, a second water-soluble polymer capable of forming two aqueous phases with the first water-soluble polymer, and a surfactant, and keeping the aqueous solution still to let the solution separate into two aqueous phases, and separating the nucleic acid and the other components in different phases.

The invention as defined in Claim 2 of the present application provides a particularly preferable automatable method of isolating nucleic acid in a sample, which comprises preparing an aqueous solution (a first polymer aqueous solution) containing a first water-soluble polymer and an aqueous solution (a second polymer aqueous solution) containing a second water-soluble polymer capable of forming two aqueous phases with the first water-soluble polymer, at least one of which contains the sample and a surfactant, bringing the first polymer aqueous solution and the second polymer aqueous solution into contact, and separating the nucleic acid and the other components in different aqueous solutions. The invention as defined in Claim 3 of the present application provides the method according to Claim 2, wherein first polymer aqueous solution and the second polymer aqueous solution are brought into contact in the form of laminar flows.

The invention as defined in Claim 4 of the present application provides the method according to Claim 1 or 2, wherein the first water-soluble polymer is polyethylene glycol, and the second water-soluble polymer is dextran.

The invention as defined in Claim 5 of the present application provides the method according to Claim 1 or 2, wherein the surfactant is a nonionic surfactant. The invention as defined in Claim 6 of the present application provides the method according to Claim 5, wherein the final concentration of the nonionic surfactant is at least 0.05%.

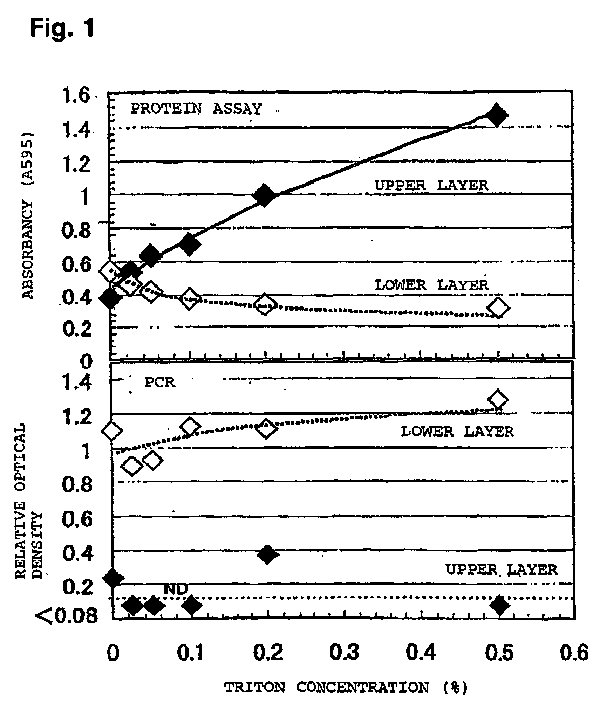

Fig. 1 shows the results of Example 1, namely the absorbencies of the recovered aqueous solutions mixed with a protein assay reagent at 595 nm and the relative densities (optical densities) of the bands of the electrophoresed PCR products (based on that of a control band obtained by electrophoresis of the PCR product from 10⁶ copies of HCV DNA). The abscissa indicates the final concentration of Triton x-100 in the two-phase aqueous system. ND denotes a undetectable PCR product which gave a band with a relative optical density of less than 0.8.

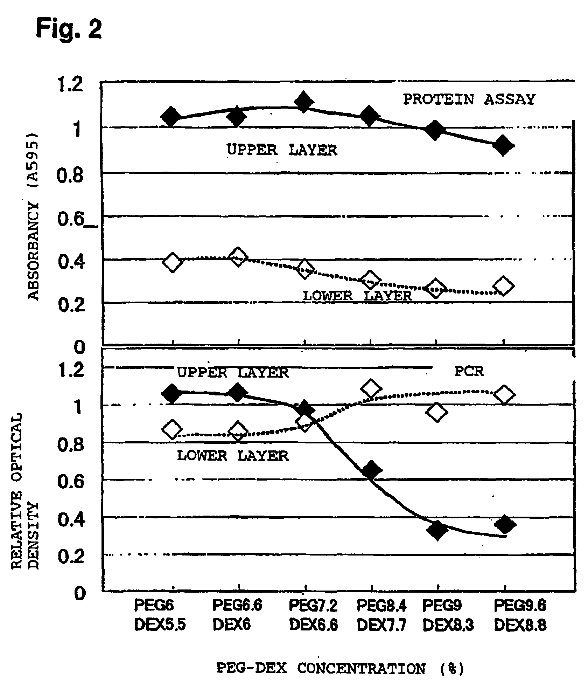

Fig. 2 shows the results of Example 2, namely the absorbencies of the recovered aqueous solutions mixed with a protein assay reagent at 595 nm and the relative densities (optical densities) of the bands of the electrophoresed PCR products (based on that of a control band obtained by electrophoresis of the PCR product from 10⁶ copies of HCV DNA). The abscissa indicates the final concentration of polyethylene glycol (PEG) or dextran (DEX) in the two-phase aqueous system.
Fig. 3

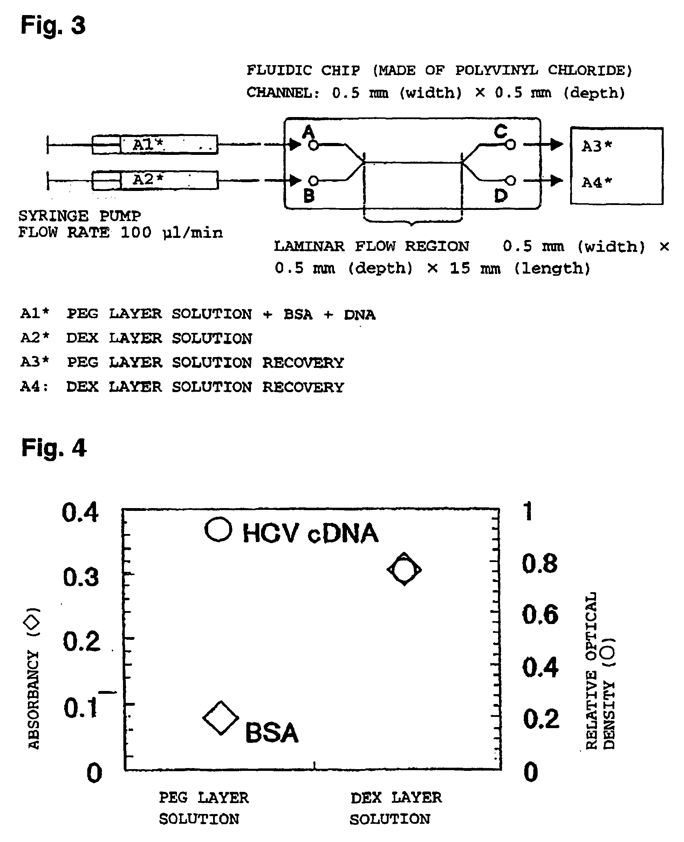

Fig. 3 shows a fluidic chip used for partitioning between laminar flows of two phases. The openings A,B,C and D lead to the channels, and in Example 3, the PEG layer solution and the DEX layer solution were introduced from A and B, respectively (flow rate: 100 µl/min) to form laminar flows in the laminar flow region. The PEG layer solution and the DEX layer solution were brought into contact in the form of laminar flows, and then the respective aqueous solutions were recovered from C and D.

Fig. 4 shows the results of Example 4, namely the absorbencies of the aqueous solutions recovered from the laminar flows of two aqueous phases mixed with a protein assay reagent at 595 nm and the relative densities (optical densities) of the bands of the electrophoresed PCR products (based on that of a control band obtained by electrophoresis of the PCR product from 10⁶ copies of HCV DNA).

Now, the present invention will be described in detail.

The nucleic acid to be isolated (prepared) by the present invention is a single-stranded or double-stranded RNA or DNA, and the sample containing the nucleic acid is a sample containing such a nucleic acid such as a reaction solution obtained after elongation, amplification, modification or other reactions of a nucleic acid, biogenic components such as serum, plasma and urea, or a crude or partly purified viral, bacterial or cellular homogenate obtained by any homogenization method without any particular restrictions.

In the present invention, firstly, an aqueous solution comprising a sample, a first water-soluble polymer, a second water-soluble polymer capable of forming two aqueous phases with the first water-soluble polymer and a surfactant is prepared. There is no particular restriction on the first and second water-soluble polymers as long as they form two aqueous phases with each other. If it is not sure whether the water-soluble polymers to be used form two aqueous phases with each other or whether they form two aqueous phases with each other at given concentrations though they certainly can form two aqueous phases, their aqueous solutions may be preliminarily prepared to check if they form two aqueous phases, and then the two aqueous phases recovered from the upper and lower layers may be used.

As the first and second water-soluble polymers, for example, polyethylene glycol, dextran, ficoll and their derivatives may be mentioned. Among them, polyethylene glycol and dextran, which readily form two aqueous phases, are preferably used as the first and second water-soluble polymers, respectively. In the case of the preferable combinations of polyethylene glycol and dextran as the first and second water-soluble polymers, the combination of a polyethylene glycol having an average molecular weight of from 3000 to 10000 with a dextran having an average molecular weight of from 100,000 to 2,000,000 is particularly preferable. In this case, the final concentrations of the polyethylene glycol and the dextran in the aqueous solution comprising a sample, the first water-soluble polymer, the second water-soluble polymer and a surfactant are preferably at least 8%, particularly preferably from 8% to 15%, in view of handling. Needless to say, polyethylene glycol and dextran may be kept in stock in the form of aqueous solutions preliminarily prepared at higher concentrations than the final concentrations.

The surfactant may be selected arbitrarily, for example, in view of the fluidity of the sample and the effect on the subsequent steps, namely, the inhibitory effect on nucleic acid amplification in the case where the isolated nucleic acid is to be amplified, and it is preferably a nonionic surfactant showing little influence during nucleic acid amplification. Such preferable nonionic surfactants include, for example, polyoxyethylene octyl phenyl ethers such as Triton x-100 and polyoxyethylene sorbitan monolaurates such as Tween 20 without any particularly restrictions. More than one surfactant may be used in combination. In the present invention, it is particularly preferred to use a nonionic surfactant at a final concentration of at least 0.05% in an aqueous solution comprising a sample, the first and second water-soluble polymers and the surfactant.

For preparation of an aqueous solution comprising a sample, the first water-soluble polymer, the second water-soluble polymer capable of forming two aqueous phases with the first water-soluble polymer and a surfactant, water or a buffer may be used, if necessary. The aqueous solution may be prepared by any method without any restrictions on the order in which the sample and the respective reagents are mixed, as long as the aqueous solution is eventually obtained, for example, by mixing them all and then adding water, by mixing a sample and the first water-soluble polymer into an aqueous solution and then adding the surfactant and the second water-soluble polymer, or by adding a sample, the surfactant and the second water-soluble polymer to an aqueous solution of the first water-soluble polymer.

The resulting aqueous solution separates into two aqueous phases upon standing, and the nucleic acid and the other components including protein are distributed to different phases. For example, when the first and second water-soluble polymers are polyethylene glycol and dextran, and the surfactant is a nonionic surfactant, two aqueous phases are formed as a polyethylene glycol-dominant upper layer (hereinafter referred to as a polyethylene glycol layer) and a dextran-dominant lower layer (hereinafter referred to as a dextran layer), and most of the nucleic acid is distributed to the dextran layer. Therefore, nucleic acid is obtained as desired by recovering the dextran layer. The phase containing nucleic acid may, if possible, be directly used in nucleic acid amplification or detection after separation, or the nucleic acid may be recovered from the phase, if necessary, by a known method.

The present invention also provides a particularly preferable automatable method of isolating nucleic acid. In this embodiment, firstly, an aqueous solution (a first polymer aqueous solution) containing a first water-soluble polymer and an aqueous solution (a second polymer aqueous solution) containing a second water-soluble polymer capable of forming two aqueous phases with the first water-soluble polymer are prepared. As the first water-soluble polymer and the second water-soluble polymer, those as previously mentioned may be mentioned. In this embodiment, the combination of polyethylene glycol and dextran as the first and second water-soluble polymers is preferable. The concentrations of the first water-soluble polymer and the second water-soluble polymer in the first polymer aqueous solution and the second polymer aqueous solution are not particularly restricted as long as these aqueous solutions form two aqueous phases upon mixing. In the case of the preferable combinations of polyethylene glycol and dextran as the first and second water-soluble polymers, it is preferred to preliminarily mix polyethylene glycol and dextran into a two-phase aqueous system having final polyethylene glycol and dextran concentrations of at least 8% as previously mentioned and then recover the polyethylene glycol layer and the dextran layer from the two-phase aqueous system for use as the respective aqueous solutions.

In the present invention, at least one of the first polymer aqueous solution and the second polymer aqueous solution contains both the sample and a surfactant, and neither solution can contain the sample or the surfactant singly. A the surfactant, as previously mentioned, a nonionic surfactant, especially a polyoxyethylene octyl phenyl ether such as Triton X-100 or a polyoxyethylene sorbitan monolaurate such as Tween 20 is preferable. When a nonionic surfactant is used, it is particularly preferred to use a nonionic surfactant at a final concentration of at least 0.05% in an aqueous solution.

By bringing the first polymer solution and the second polymer solution into contact, if necessary with stirring, and then keeping the resulting aqueous solution still to let the aqueous solution form two aqueous phases, nucleic acid and the other components such as protein get distributed to different phases. In the present invention, the first polymer aqueous solution and the second polymer aqueous solution are brought into contact in the form of laminar flows which run in flow paths such as channels or pipes. Such laminar flows of the first polymer aqueous solution and the second polymer aqueous solution can be brought into contact in microchannels cut in a substrate by a known method such as photolithography, and the use of microchannels contributes to fast nucleic acid distribution, the reduction in size and cost of the instruments and the substrate required to carry out the invention and automation of the present invention.

For example, in a preferable case where laminar flows of a polyethylene glycol aqueous solution and a dextran aqueous solution as the first and second polymer aqueous solutions are brought into contact in microchannels, most of the protein contaminant is distributed to the polyethylene glycol layer, while nucleic acid is distributed to the dextran layer. Therefore, nucleic acid is obtained as desired by recovering the dextran layer. The phase containing nucleic acid may, if possible, be directly used in nucleic acid amplification or detection after separation, or the nucleic acid may be recovered from the phase, if necessary, by a known method.

Now, the present invention will be described in further detail by referring to Examples. However, the present invention is by no means restricted to these specific Examples.

### EXAMPLE 1 Separation of protein and nucleic acid using partition between two aqueous phases containing surfactants

(1) To 1 ml of aqueous solutions containing polyethylene glycol 6000 (average molecular weight 7500) at a final concentration of 9%, dextran (molecular weight 480,000) at a final concentration of 8.5% and Triton x-100 at final concentrations of from 0 to 0.5%, 100 µg of bovine serum albumin (hereinafter referred to as "BSA") and 5×10⁷ copies of a double-stranded DNA (hereinafter referred to as "HCV DNA") containing HCV (hepatitis C virus) cDNA (bases 1 to 1865 (for the sequence and base numbers, Kato, N et al., Proc, Natl. Acad. Sci. USA (1990), 87, 9524-9528 should be referred to) were added and mixed.
(2) The resulting aqueous solutions were kept still so as to separate into two phases, and the upper layer (dominated by polyethylene glycol) and the lower layer (dominated by dextran) were recovered, respectively.
(3) To 50 µl portions of the recovered aqueous solutions, 350 µl of distilled water and 100 µl of a protein assay reagent (product name, BIO-RAD PROTEIN ASSAY, Bio-Rad Lab.) was added, and the absorbancies were measured at 595 nm.
(4) Separately, the recovered aqueous solutions were diluted with TE by a factor of 10, and 10 µl of the resulting solutions were poured into PCR tubes (capacity 0.5 ml, product name, GeneAmp Thin-Walled Reaction Tubes, Perkin Elmer).
   The composition of TE
   10 mM Tris-HCl (pH 8.0)
   0.1 mM EDTA
(5) Then, 65 µl of a PCR mix (having the following composition) was added, and 60 µl of mineral oil was laid over. PCR was carried out in a thermal cycler (product name, GeneAmp 9600 PCR system, Perkin Elmer) under the following conditions.
   The composition of the PCR mix
   11.5 mM Tris-HCl (pH 8.3)
   57.7 mM KCl
   2.6 mM MgCl₂
   0.3 mM dNTP (dATP, dGTP, dCTP and dTTP each 0.3 mM)
   0.28 mM Primer (R) (complementary to bases 248 to 267 of HCV cDNA (Kato et al.), SEQ ID NO:1)
   0.28 mM Primer (F) (homologous to bases 10 to 31 of HCV cDNA (Kato et al., SEQ ID NO:2)
   0.023% Triton X-100
   0.03S U/µl AmpliTaq Gold (product name, Perkin Elmer Japan)
   PCR conditions
   ① 95 °C, 9 minutes
   ② 95 °C, 30 seconds
   ③ 62°C, 30 seconds
   ④ 72°C, 1 minute
   the 40 cycles of ② to ④
(6) The PCR products were electrophoresed on a 4% agarose gel and stained with SYBR Green II (product name, Takara Shuzo Co., Ltd.). The densities of the bands obtained by the electrophoresis were measured with a densitometer (product name, densitograph, ATTO Corporation).

The absorbencies measured with the protein assay reagent in (3) and the densities of the electrophoretically obtained bands measured in (6) were shown in Fig. 1. In the presence of at least 0.05% of Triton X-100, HCV DNA was mostly distributed to the lower layer, while BSA was mostly distributed to the upper layer. Thus, in the presence of at least 0.05% of Triton X-100, HCV DNA and BSA could be separated.

### EXAMPLE 2 Separation of protein and nucleic acid using partition between two aqueous phases

(1) To 1 ml of aqueous solutions containing polyethylene glycol 6000 (average molecular weight 7500) at final concentrations of from 6 to 9.6%, dextran (molecular weight 480,000) at final concentrations of from 5.5% to 8.8% and Triton X-100 at a final concentration of from 0.1%, 100 µg of BSA and 5×10⁷ copies of HCV DNA were added and mixed.
(2) The resulting aqueous solutions were kept still so as to separate into two phases, and the upper layer (dominated by polyethylene glycol) and the lower layer (dominated by dextran) were recovered, respectively.
(3) To 100 µl portions of the recovered aqueous solutions, 500 µl of distilled water and 100 µl of a protein assay reagent (product name, BIO-RAD PROTEIN ASSAY, Bio-Rad Lab.) were added, and the absorbancies were measured at 595 nm.
(4) Separately, the recovered aqueous solutions were diluted with TE by a factor of 10, and 10 µl of the resulting solutions were poured into PCR tubes (capacity 0.5 ml, product name, GeneAmp Thin-Walled Reaction Tubes, Perkin Elmer) .
(5) Then, 65 µl of a PCR mix (which is the same as the one used in Example 1) was added, and 60 µl of mineral oil was laid over. PCR was carried out in a thermal cycler (product name, GeneAmp 9600 PCR system, Perkin Elmer) under the same conditions as in Example 1.
(6) The PCR products were electrophoresed on a 4% agarose gel and stained with SYBR Green II (product name, Takara Shuzo Co., Ltd.) The densities of the bands obtained by the electrophoresis were measured with a densitometer (product name, densitograph, ATTO Corporation).

The absorbencies measured with the protein assay reagent in (3) and the densities of the electrophoretically obtained bands measured in (6) were shown in Fig. 2. HCV DNA was mostly distributed to the upper layer in the presence of at most 7.2% of polyethylene glycol and at most 6.6% of dextran, and to the lower layer in the presence of at lest 8.4% of polyethylene glycol and at least 7.7% of dextran, while BSA was mostly distributed to the upper layer irrespective of their concentrations. Thus, in the presence of Triton X-100, when the polyethylene glycol concentration and the dextran concentration were at lest about 8%, HCV DNA and BSA could be separated.

### EXAMPLE 3 Partition between laminar flows of two aqueous phases

(1) 2 ml of an aqueous solution containing polyethylene glycol 6000 (average molecular weight 7500) at final concentrations of 11.3% and dextran at a final concentration of 10.6% was stirred and then kept still to separate into two phases.
(2) The two phases in the upper layer (hereinafter referred to as the PEG layer) and the lower layer (hereinafter referred to as the DEX layer) were recovered, respectively.
(3) To 0.8 ml of the DEX layer solution, 0.2% of Triton X-100 was added, and 100 µg of BSA and 5×10⁷ copies of HCV DNA were added to a total volume of 1 ml. On the other hand, to 0.8 ml of the PEG layer solution, 0.2 ml of distilled water was added to a total volume of 1 ml.
(4) The PEG layer solution and the DEX layer solution were introduced into a fluidic chip shown in Fig. 3 through the openings A and B, respectively, by means of syringe pumps at flow rates of 100 µl/min so as to form laminar flows and then recovered from the openings c and D.
(5) To 50 µl of the recovered aqueous solutions, 350 µl of distilled water and 100 µl of a protein assay reagent (product name, BIO-RAD PROTEIN ASSAY, Bio-Rad Lab.) were added, and the absorbancies were measured at 595 nm.
(6) Separately, the recovered aqueous solutions were diluted with TE by a factor of 10, and 10 µl of the resulting solutions were poured into PCR tubes (capacity 0.5 ml, product name, GeneAmp Thin-Walled Reaction Tubes, Perkin Elmer).
(7) Then, 65 µl of a PCR mix (which is the same as the one used in Example 1) was added, and 60 µl of mineral oil was laid over. PCR was carried out in a thermal cycler (product name, GeneAmp 9600 PCR system, Perkin Elmer) under the same conditions as in Example 1.
(8) The PCR products were electrophoresed on a 4% agarose gel and stained with SYBR Green II (product name, Takara Shuzo Co., Ltd.). The densities of the bands obtained by the electrophoresis were measured with a densitometer (product name, densitograph, ATTO Corporation).

The absorbencies measured with the protein assay reagent in (3) and the densities of the electrophoretically obtained bands measured in (8) were shown in Fig. 4. HCV DAN was distributed to the DEX layer solution, while BSV was distributed to the PEG layer solution. Thus, partitioning between laminar flows of two phases in a fluidic ship shown in Fig. 3 affords fast separation of HCV DNA and BSA (100 µl of the aqueous solution containing nucleic acid was recovered within about 1 minute).

As described above, the present invention provides a method of isolating (preparing) nucleic acid which neither uses deleterious substances such as phenol and chloroform nor requires a special instrument such as filtration and centrifugation, and therefore is easy to automate. The present invention can also be carried out in microchannels in microplates or integrated plates. Therefore, the present invention provides an important method which contributes to provision of microplates which isolate nucleic acid to be amplified for detection and quantification of certain nucleic acids.

## Claims

1. A method of isolating nucleic acid in a sample, which comprises preparing an aqueous solution comprising the sample, a first water-soluble polymer, a second water-soluble polymer capable of forming two aqueous phases with the first water-soluble polymer, and a surfactant, and keeping the aqueous solution still to let the solution separate into two aqueous phases, and separating the nucleic acid and the other components in different phases.

2. A method of isolating nucleic acid in a sample, which comprises preparing an aqueous solution (a first polymer aqueous solution) containing a first water-soluble polymer and an aqueous solution (a second polymer aqueous solution) containing a second water-soluble polymer capable of forming two aqueous phases with the first water-soluble polymer, at least one of which contains the sample and a surfactant, bringing the first polymer aqueous solution and the second polymer aqueous solution into contact, and separating the nucleic acid and the other components in different aqueous solutions.

3. The method of isolating nucleic acid according to Claim 2, wherein the first polymer aqueous solution and the second polymer aqueous solution are brought into contact in the form of laminar flows.

4. The method according to Claim 1 or 2, wherein the first water-soluble polymer is polyethylene glycol, and the second water-soluble polymer is dextran.

5. The method according to Claim 1 or 2, wherein the surfactant is a nonionic surfactant.

6. The method according to Claim 5, wherein the final concentration of the nonionic surfactant is at least 0.05%.

## Patentansprüche

1. Verfahren für die Isolierung von Nukleinsäure in einer Probe, welches die Herstellung einer wässrigen Lösung mit der Probe, einem ersten wasserlöslichen Polymer, einem zweiten wasserlöslichen Polymer, das zwei wässrige Phasen mit dem ersten wasserlöslichen Polymer bilden kann, und einem oberflächenaktiven Stoff umfasst, und Stillhalten der wässrigen Lösung, damit sich die Lösung in zwei wässrige Phasen trennt, und Trennen der Nukleinsäure und der anderen Bestandteile in unterschiedlichen Phasen.

2. Verfahren für die Isolierung von Nukleinsäure in einer Probe, welches die Herstellung einer wässrigen Lösung (einer ersten wässrigen Polymerlösung) mit einem ersten wasserlöslichen Polymer und einer wässrigen Lösung (einer zweiten wässrigen Polymerlösung), die ein zweites wasserlösliches Polymer enthält, das zwei wässrige Phasen mit dem ersten wasserlöslichen Polymer bilden kann, umfasst, wobei wenigstens eines davon die Probe und einen oberflächenaktiven Stoff umfasst, in Kontakt bringen der ersten wässrigen Polymerlösung und der zweiten wässrigen Polymerlösung, und Trennen der Nukleinsäure und der anderen Bestandteile in unterschiedliche wässrige Lösungen.

3. Verfahren für die Isolierung von Nukleinsäure nach Anspruch 2, wobei die erste wässrige Polymerlösung und die zweite wässrige Polymerlösung in der Form von laminaren Strömungen in Kontakt gebracht werden.

4. Verfahren nach Anspruch 1 oder 2, wobei das erste wasserlösliche Polymer Polyethylenglycol und das zweite wasserlösliche Polymer Dextran ist.

5. Verfahren nach Anspruch 1 oder 2, wobei der oberflächenaktive Stoff ein nichtionischer oberflächenaktiver Stoff ist.

6. Verfahren nach Anspruch 5, wobei die Endkonzentration des nichtionischen oberflächenaktiven Stoffs wenigstens 0,05% ist.

## Revendications

1. Procédé d'isolation d'acide nucléique dans un échantillon, qui comprend de préparer une solution aqueuse comprenant l'échantillon, un premier polymère soluble dans l'eau, un second polymère soluble dans l'eau capable de former deux phases aqueuses avec le premier polymère soluble dans l'eau, et un tensioactif, et de mettre de côté la solution aqueuse afin de laisser la solution se séparer en deux phases aqueuses, et de séparer l'acide nucléique et les autres composants dans des phases différentes.

2. Procédé d'isolation d'acide nucléique dans un échantillon, qui comprend de préparer une solution aqueuse (une première solution aqueuse de polymère) contenant un premier polymère soluble dans l'eau et une solution aqueuse (une seconde solution aqueuse de polymère) contenant un second polymère soluble dans l'eau capable de former deux phases aqueuses avec le premier polymère soluble dans l'eau, dont au moins l'une de celles-ci contient l'échantillon et un tensioactif, de mettre la première solution aqueuse de polymère et la seconde solution aqueuse de polymère en contact, et de séparer l'acides nucléique et les autres composants dans des solutions aqueuses différentes.

3. Procédé d'isolation d'acide nucléique selon la revendication 2, dans lequel la première solution aqueuse de polymère et la seconde solution aqueuse de polymère sont mises en contact sous la forme d'écoulements laminaires.

4. Procédé selon la revendication 1 ou 2, dans lequel le premier polymère soluble dans l'eau est un polyéthylène glycol, et le second polymère soluble dans l'eau est un dextran.

5. Procédé selon la revendication 1 ou 2, dans lequel le tensioactif est un tensioactif non ionique.

6. Procédé selon la revendication 5, dans lequel la concentration finale du tensioactif non ionique est au moins 0,05 %.
